# FASCICULE DE BREVET EUROPEEN

(11) **EP 3 179 929 B1**
(45) Date de publication et mention de la délivrance du brevet: **08.01.2020**
(21) Numéro de dépôt: 15766897.1
(22) Date de dépôt: 07.08.2015
(51) Int. Cl.: A61B 17/88, B25G 1/00, A61B 17/3211, B25G 1/06, B25B 15/02, B25G 1/08, A61B 17/00, A61B 17/28

(54) **MANCHE DE PREHENSION POUR OUTIL CHIRURGICAL, PROCEDE ET MACHINE DE FABRICATION D'UN TEL MANCHE DE PREHENSION**
HANDGRIFF FÜR CHIRURGISCHES WERKZEUG UND VERFAHREN UND MASCHINE ZUR HERSTELLUNG EINES SOLCHEN HANDGRIFF
GRIPPING HANDLE FOR SURGICAL TOOL AND METHOD AND MACHINE FOR MAKING SUCH A GRIPPING HANDLE

(30) Priorité: 13.08.2014 FR 1457781
(43) Date de publication de la demande: 21.06.2017
(73) Titulaire: In2Bones, 69130 Ecully (FR)
(72) Inventeur: COILLARD-LAVIROTTE, Jean-Yves Paul Albert, 69450 Saint Cyr Au Mont D'Or (FR); BOUBLIL, Daniel Edmond, 69006 Lyon (FR); D'INGRADO, Philippe Emmanuel, 69370 Saint Didier Au Mont D'Or (FR)
(74) Mandataire: Weber, Jean-François
(86) Numéro de dépôt international: PCT/FR2015/052177
(87) Numéro de publication internationale: WO 2016/024063

(56) Documents cités:
- US-A1- 2005 178 251
- US-A1- 2014 121 694

## Description

### DOMAINE TECHNIQUE

La présente invention concerne le domaine de l'instrumentation chirurgicale, en particulier des outils chirurgicaux à manches amovibles ou interchangeables, tels que les tournevis chirurgicaux à embouts.

L'invention concerne plus précisément un manche de préhension conçu pour recevoir un embout de travail amovible afin de former un outil chirurgical avec ce dernier, ledit manche de préhension comprenant :
- un corps principal s'étendant le long d'un axe longitudinal entre une extrémité proximale et une extrémité distale,
- un orifice de réception ménagé au sein du corps principal et étant conçu pour recevoir l'embout de travail à coulissement.

L'invention concerne également un kit d'outil chirurgical comprenant un manche de préhension.

L'invention concerne en outre un procédé de fabrication d'un manche de préhension.

L'invention concerne enfin une machine de fabrication.

### TECHNIQUE ANTERIEURE

Dans le domaine de l'instrumentation pour la chirurgie, on connaît des tournevis chirurgicaux modulables, à embouts interchangeables. Un chirurgien peut ainsi, équipé d'un unique manche de tournevis et d'une pluralité d'embouts de tournevis aux fonctions différentes, faire face à une multitude de situations chirurgicales.

Pour ces tournevis modulables connus, il est nécessaire, lors de l'assemblage du manche avec un embout donné, que cet assemblage soit particulièrement fiable et solide, en particulier pour qu'il ne subsiste aucun risque de décrochage accidentel de l'embout, et pour que le jeu entre le manche et ledit embout soit réduit au maximum, compte-tenu de la précision nécessaire pour les gestes chirurgicaux.

Par conséquent, ces tournevis connus sont généralement fabriqués à l'aide de pièces dont la précision est élevée, et qui font l'objet d'un assemblage complexe et précis pour former le manche d'un tournevis présentant les qualités requises, de sorte qu'ils sont relativement coûteux et difficiles à fabriquer. Par ailleurs, la nécessité de nettoyer et stériliser l'instrumentation chirurgicale entre chaque opération est susceptible de détériorer plus ou moins progressivement cet assemblage, de sorte que l'outil connu peut présenter un jeu, ou une usure progressive.

Pour les tournevis modulables connus, la solidarisation d'un embout donné au manche amovible peut être effectuée en actionnant une pièce mobile du manche, par exemple un bouton ou un levier rotatif. L'usure de telles pièces peut éventuellement conduire à rendre leur actionnement malaisé, ou au contraire trop souple, de sorte que le changement d'embout est susceptible de devenir difficile au cours du temps, ce qui est susceptible de causer une perte de temps substantielle au cours d'une opération chirurgicale, ou au contraire trop lâche, de manière à augmenter le risque de désolidarisation accidentelle de l'embout.

Le document US 2014/121694 A1 décrit un manche de préhension selon le préambule de la revendication 1.

### EXPOSE DE L'INVENTION

Les objets assignés à la présente invention visent en conséquence à remédier aux différents inconvénients énumérés précédemment et à proposer un nouveau manche de préhension, un nouveau kit d'outil chirurgical, un nouveau procédé de fabrication et une nouvelle machine de fabrication, permettant une fabrication particulièrement rapide et peu coûteux à fabriquer.

Un autre objet de l'invention vise à proposer un nouveau manche de préhension, un nouveau kit d'outil chirurgical, un nouveau procédé de fabrication et une nouvelle machine de fabrication permettant de former un outil chirurgical particulièrement fiable et robuste.

Un autre objet de l'invention vise à proposer un nouveau manche de préhension, un nouveau kit d'outil chirurgical un nouveau procédé de fabrication et une nouvelle machine de fabrication, permettant de former un manche de préhension dont l'utilisation est particulièrement aisée et confortable.

Un autre objet de l'invention vise à proposer un nouveau manche de préhension, un nouveau kit d'outil chirurgical, un nouveau procédé de fabrication et une nouvelle machine de fabrication, permettant de former un manche de préhension particulièrement facile à fabriquer.

Un autre objet de l'invention vise à proposer un nouveau manche de préhension, un nouveau kit d'outil chirurgical, un nouveau procédé de fabrication et une nouvelle machine de fabrication, permettant de former un manche de préhension amovible destiné à appartenir à un outil chirurgical d'une grande précision et sensiblement dépourvu de jeu de montage.

Un autre objet de l'invention vise à proposer un nouveau manche de préhension et un nouveau kit d'outil chirurgical permettant de garantir une bonne stérilisation de l'environnement chirurgical.

Les objets assignés à l'invention sont atteints à l'aide d'un manche de préhension conçu pour recevoir un embout de travail amovible afin de former un outil chirurgical avec ce dernier, ledit manche de préhension comprenant :
- un corps principal s'étendant le long d'un axe longitudinal entre une extrémité proximale et une extrémité distale,
- un orifice de réception ménagé au sein du corps principal et étant conçu pour recevoir l'embout de travail à coulissement,
   ledit manche de préhension étant caractérisé en ce qu'il comprend
- un levier de blocage du coulissement de l'embout de travail dans l'orifice de réception, le levier de blocage étant monté à rotation sur le corps principal autour d'un axe de rotation de manière à pouvoir être basculé entre :
   ∘ une orientation de blocage dans laquelle ledit levier bloque le coulissement de l'embout de travail,
   ∘ une orientation de libération dans laquelle il autorise le coulissement de l'embout de travail dans l'orifice de réception,
- un pivot élastique par l'intermédiaire duquel le levier de blocage est monté à rotation sur le corps principal, le pivot élastique étant conçu pour ramener de lui-même le levier de blocage en orientation de blocage lorsque ce dernier est en orientation de libération,
et en ce que le corps principal, le levier de blocage et le pivot élastique viennent de matière les uns avec les autres de manière à former une pièce d'un seul tenant.

Les objets assignés à l'invention sont également atteints à l'aide d'un kit d'outil chirurgical comprenant un manche de préhension selon l'invention, ainsi qu'au moins un embout de travail amovible, de préférence deux embouts de travail amovibles de fonction différente.

Les objets assignés à l'invention sont en outre atteints à l'aide d'un procédé de fabrication d'un manche de préhension selon l'invention, le procédé de fabrication étant caractérisé en ce qu'il comprend une unique étape de moulage au cours de laquelle on réalise le manche de préhension d'un seul tenant dans son intégralité.

Enfin, les objets assignés à l'invention sont atteints à l'aide d'une machine de fabrication d'un manche de préhension conforme à ce qui précède, ladite machine de fabrication étant caractérisée en ce qu'elle comprend un moule comprenant une première empreinte de moule, une deuxième empreinte de moule et au moins un premier tiroir, lesquels sont destinés à former un espace clos dans lequel un matériau prévu pour former le manche de préhension est destiné à être coulé, la première empreinte de moule et la deuxième empreinte de moule étant conçues pour modeler conjointement le matériau afin de former, au moins en partie, le corps principal, le levier de blocage et le pivot élastique, le premier tiroir étant conçu pour ménager l'orifice de réception au sein du corps principal, ladite machine de fabrication permettant de mettre en œuvre le procédé de fabrication susvisé.

### DESCRIPTIF SOMMAIRE DES DESSINS

D'autres particularités et avantages de l'invention apparaîtront et ressortiront plus en détails à la lecture de la description faite ci-après, en référence aux dessins annexés, donnés uniquement à titre d'exemple illustratif et non limitatif, dans lesquels :
- La figure 1 illustre, selon une vue en perspective générale, un manche de préhension selon l'invention,
- La figure 2 représente, selon une vue en perspective générale du dessus, le manche de préhension de la figure 1,
- La figure 3 illustre, selon une vue en coupe longitudinale en perspective, le manche de préhension des figures 1 et 2,
- La figure 4 illustre, selon une vue partielle en perspective, un embout de travail amovible selon l'invention, destiné à être assemblé avec le manche de préhension des figures 1 à 3 pour former un outil chirurgical,
- Les figures 5 et 6 illustrent, selon des vues en coupe longitudinale en perspective, le manche de préhension des figures 1 à 3 associé avec l'embout de travail amovible de la figure 4,
- La figure 7 représente un détail 6 bis de réalisation du manche de préhension des figures 1 à 3,
- La figure 8 représente une vue générale en perspective de l'embout de travail des figures 4 à 6,
- Les figures 9 et 10 illustrent chacune une vue générale en perspective d'une variante distincte d'embout de travail associable au manche de préhension des figures 1 à 7.
- La figure 11 illustre, selon une vue en perspective éclatée de côté, une autre variante d'un manche de préhension et d'un embout de travail conformes à l'invention,
- Les figures 12 et 13 illustrent, selon des vues en perspective, le manche de préhension et l'embout de travail de la figure 10, le manche de préhension ayant été coupé le longitudinalement.

### MEILLEURE MANIERE DE REALISER L'INVENTION

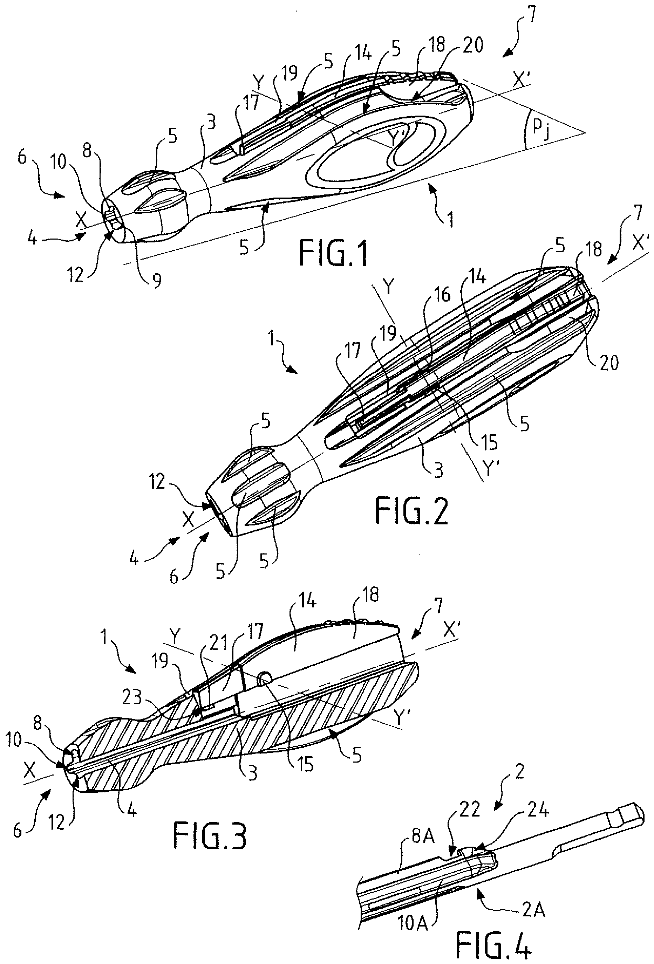

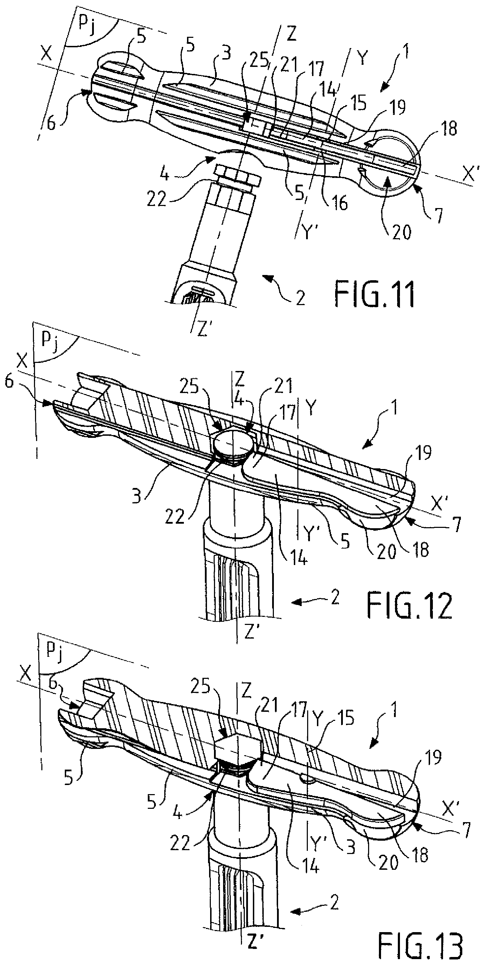

L'invention concerne une pièce destinée à contribuer à former un outil chirurgical démontable et modulaire, et concerne en l'espèce un manche de préhension 1 de l'outil chirurgical dont un premier exemple de réalisation conforme à l'invention est illustré aux figures 1 à 3, et un deuxième exemple de réalisation conforme à l'invention est illustré aux figures 11 à 13.

Le manche de préhension 1, qui constitue en d'autres termes une poignée de préhension ou encore un pommeau de préhension, est conçu, selon l'invention, pour recevoir un embout de travail 2 amovible afin de former un outil chirurgical avec ce dernier, c'est-à-dire en l'espèce un instrument chirurgical particulièrement adapté à être utilisé par un chirurgien au cours d'une opération chirurgicale sur un patient. Bien entendu, sans sortir du cadre de l'invention, le manche de préhension 1 et l'outil chirurgical qu'il contribue à former pourront être utilisés dans le cadre d'une chirurgie animale, ou pour des utilisations étrangères à la chirurgie, par exemple pour effectuer le vissage d'une vis au sein d'un mécanisme non vivant.

Le manche de préhension 1 de l'invention constitue une zone de l'outil chirurgical formé qui peut être saisie par le chirurgien de façon privilégiée, par exemple à une main, ou deux. L'embout de travail 2, quant à lui, forme la partie fonctionnelle de l'outil, et constitue avantageusement un moyen d'action, par exemple mécanique, sur le corps du patient, ou sur un autre objet, par exemple une vis ou une agrafe. De manière avantageuse, l'instrument chirurgical ainsi formé pourra être utilisé par le chirurgien, en fonction de l'embout de travail 2 reçu par le manche de préhension 1, pour effectuer :
- le vissage ou le dévissage d'une vis, par exemple d'ostéosynthèse, ou
- la mise en place d'une agrafe d'ostéosynthèse, dans le corps d'un patient.

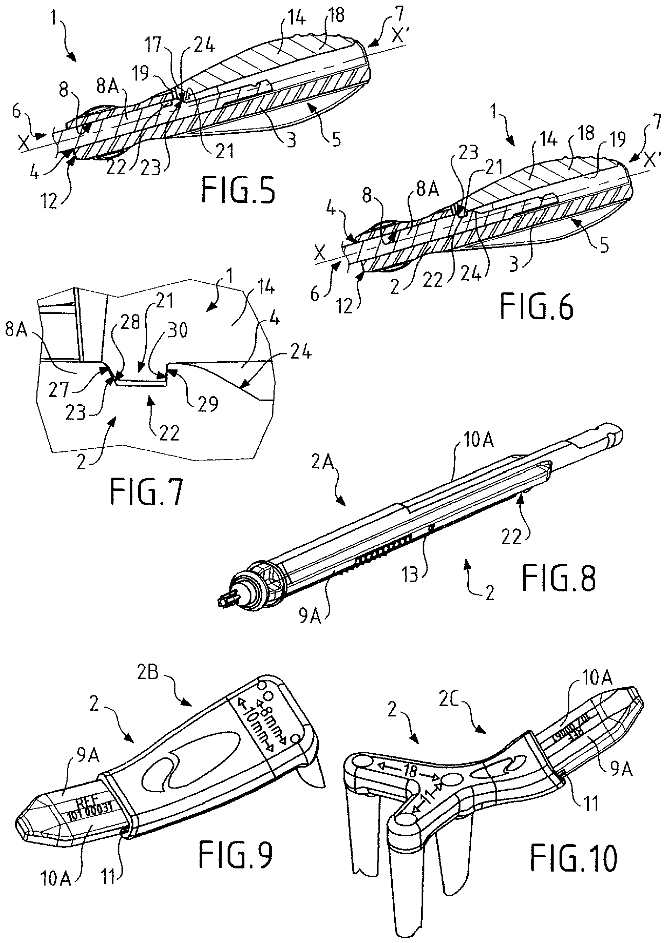

Pour effectuer les actions qui précèdent, le chirurgien pourra utiliser plusieurs variantes distinctes d'embouts de travail, en particulier :
- un embout de travail 2 formant un embout de tournevis 2A, c'est-à-dire un axe de tournevis (tel qu'illustré à la figure 8),
- un embout de travail 2 formant un guide de mise en place de broches 2B (tel qu'illustré à la figure 9),
- un embout de travail 2 formant un guide de perçage 2C pour la mise en place d'une agrafe (tel qu'illustré à la figure 10),
- un embout de travail 2 formant une lame de scalpel (non représenté).

Le manche de préhension 1, quant à lui, forme avantageusement un manche de tournevis, et/ou un manche de support de guide de mise en place de broches et/ou de guide de perçage, et/ou un manche de scalpel. De préférence, le manche de préhension 1 est multifonction et polyvalent, et est adapté à recevoir des embouts de travail 2 dont les fonctions sont variées et distinctes.

Le manche de préhension 1 de l'invention comprend un corps principal 3 s'étendant le long d'un axe longitudinal X-X' entre une extrémité proximale 7 et une extrémité distale 6. Le corps principal 3 présente avantageusement une forme générale axiale ou allongée, de préférence une forme générale de révolution autour de l'axe longitudinal X-X', de manière à être adapté à être pris en main par le chirurgien de manière ergonomique et sûre.

Le corps principal 3 présente préférentiellement la forme d'un manche de tournevis classique, tel qu'illustré par exemple aux figures 1 à 3, mais peut alternativement présenter la forme d'un pommeau ou d'une poignée, tel qu'illustré par exemple aux figures 11 à 13.

Pour l'exemple préférentiel illustré aux figures 1 à 3, de préférence, l'extrémité proximale 7 est destinée à former l'arrière de l'outil chirurgical, et plus précisément l'extrémité de l'outil qui est destinée à être tournée en direction du chirurgien. L'extrémité distale 6 sera quant à elle avantageusement destinée à être tournée en direction du patient. L'outil chirurgical formé sera ainsi préférentiellement un outil axial droit.

Dans le deuxième exemple préférentiel illustré aux figures 11 à 13, le manche de préhension 1 pourra servir à former par exemple un outil coudé ou en « *T* », l'embout de travail 2 s'étendant alors perpendiculairement au manche de préhension 1, de sorte que l'extrémité distale 6 et l'extrémité proximale 7 forment une partie droite et gauche de la poignée de préhension 1 de l'outil, laquelle est par exemple destinée à être disposée transversalement par rapport au patient

Dans le cas préférentiel ou le manche de préhension 1 est destiné à former le manche d'un tournevis, il sera destiné à être mis en rotation autour de son axe longitudinal X-X' afin d'effectuer le vissage ou le dévissage d'une vis par l'intermédiaire de l'embout de travail 2. Dans ce cas préférentiel, ou dans tout autre cas, le manche de préhension 1 présente une portion distale dont la forme générale est celle d'une ellipsoïde de révolution, dont le diamètre de révolution est coaxial avec l'axe longitudinal X-X'. Une telle forme est particulièrement adaptée à la prise en main pour rotation autour de l'axe longitudinal X-X', tel qu'illustré aux figures.

Le manche de préhension 1 pourra préférentiellement présenter une surface extérieure antidérapante. En particulier, le manche de préhension 1 pourra présenter des sillons 5, par exemple parallèles entre eux, ménagés dans le corps principal 3 le long de ce dernier, selon une direction longitudinale. Ces sillons 5 ont avantageusement la triple fonction d'améliorer l'adhérence de la préhension, d'alléger le corps principal 3 pour économiser poids et matière, et de permettre une réalisation par moulage dudit corps principal 3. En effet, les sillons 5 sont préférentiellement suffisamment profonds pour faire en sorte que le corps principal 3, tout en présentant un contour extérieur ergonomique, par exemple de la forme d'un manche de tournevis classique, soit sensiblement dénué de zones où la matière est très épaisse, afin de minimiser le retrait au démoulage. De plus, les sillons 5 sont disposés dans le corps principal 3 de manière à ce que ce dernier puisse être démoulé au cours de sa fabrication, autour d'un plan de joint Pⱼ incluant par exemple l'axe longitudinal X-X'. A cet effet, les sillons 5 sont avantageusement orientés selon des plans orthogonaux audit plan de joint Pⱼ, parallèles à l'axe longitudinal X-X', et sont par exemple en dépouille par rapport à ce dernier.

Selon l'invention, le manche de préhension 1 comprend également un orifice de réception 4 ménagé au sein du corps principal 3 et étant conçu pour recevoir l'embout de travail 2 à coulissement. Le manche de préhension 1 inclut ainsi un orifice de réception 4 au sein duquel l'embout de travail 2 peut être assemblé au manche de préhension 1. La forme, et surtout la section de l'orifice de réception 4 est avantageusement conformée pour correspondre avec l'embout de travail 2, de manière à ce que le manche de préhension 1 forme la partie femelle de l'outil chirurgical, l'embout de travail 2 formant la partie mâle.

Dans le cas des figures 1 à 3, l'orifice de réception 4 est avantageusement ménagé au sein du corps principal 3 à partir de l'extrémité distale 6 de manière à former un outil chirurgical axial. Dans ce cas, l'orifice de réception 4 est préférentiellement conçu pour recevoir l'embout de travail 2 à coulissement le long de l'axe longitudinal X-X', de manière à former un outil chirurgical axial.

Le manche de préhension 1 est conçu pour que l'embout de travail 2, étant inséré dans l'orifice de réception 4, puisse translater au sein de ce dernier dans le sens de la longueur du corps principal, selon une course limitée ou non. L'orifice de réception 4 forme ainsi un rail, ou encore un guide de coulissement de l'embout de travail 2 le long de l'axe longitudinal X-X'.

De préférence, comme illustré aux figures 1 à 3, l'orifice de réception 4 est ménagé au sein du corps principal 3 à partir de l'extrémité distale 6, par exemple de façon alignée avec l'axe longitudinal X-X'. Bien entendu, rien ne s'oppose à ce que l'orifice de réception 4 soit ménagé à partir de la surface latérale du corps principal 3, où de façon désaxée par rapport à l'axe longitudinal X-X'. L'orifice de réception 4 traverse optionnellement le corps principal 3 de part en part, par exemple de l'extrémité distale 6 à l'extrémité proximale 7, ce qui permet en particulier de faciliter la fabrication du manche de préhension 1.

Alternativement, dans le cas préférentiel représenté par exemple aux figures 11 à 13, l'orifice de réception 4 est ménagé au sein du corps principal 3, de manière à déboucher à partir de la surface externe de ce dernier, entre l'extrémité distale 6 et l'extrémité proximale 7. L'orifice de réception 4 est ainsi préférentiellement situé sur une portion intermédiaire du manche de préhension 1, et est de préférence situé à mi-chemin, de façon médiane, entre l'extrémité proximale 7 et l'extrémité distale 6.

L'orifice de réception 4 est avantageusement conçu, dans ce cas, pour recevoir l'embout de travail 2 à coulissement le long d'un axe orthogonal Z-Z' orthogonal à l'axe longitudinal X-X' de manière à former un outil chirurgical en « T ».

De préférence, l'orifice de réception 4 présente une forme congruente avec le contour extérieur de l'embout de travail 2, et permet de bloquer en rotation l'embout de travail 2 par rapport audit manche de préhension 1 respectivement autour de l'axe longitudinal X-X' pour la variante préférentielle des figures 1 à 3, ou autour de l'axe orthogonal Z-Z' pour la variante préférentielle des figures 11 à 13, tout en autorisant le coulissement dudit embout de travail 2 par rapport au manche de préhension 1 selon le même axe.

Pour permettre un entraînement en rotation de l'embout de travail 2 par le manche de préhension 1 respectivement autour de l'axe longitudinal X-X', ou de l'axe orthogonal Z-Z', l'ouverture de réception 4 présente, sur au moins une portion de sa profondeur, une forme qui n'est avantageusement pas une forme de révolution respectivement autour de l'axe longitudinal X-X', ou de l'axe orthogonal Z-Z'.

De façon préférentielle, notamment dans le cas préférentiel représenté aux figures 1 à 3, l'orifice de réception 4 présente une section dont la forme permet de bloquer en rotation l'embout de travail 2 par rapport audit manche de préhension 1 autour de l'axe longitudinal X-X', tout en autorisant le coulissement dudit embout de travail 2 par rapport au manche de préhension 1 le long de l'axe longitudinal X-X'. L'orifice de réception 4 n'autorise sensiblement ainsi, selon cette configuration, qu'un seul degré de liberté à l'embout de travail 2 lorsque ce dernier est inséré au sein dudit orifice de réception 4.

A cet effet notamment, l'orifice de réception 4 présente au moins une rainure longitudinale principale 8 s'étendant sur au moins une portion de la longueur dudit orifice de réception 4, la rainure longitudinale principale 8 étant conçue pour coopérer avec une ailette longitudinale principale 8A de l'embout de travail 2 afin de solidariser en rotation ledit embout de travail 2 par rapport audit manche de préhension 1 autour de l'axe longitudinal X-X' (tel qu'illustré aux figures 1 à 3). Ainsi, de préférence, lors de la mise en rotation autour de l'axe longitudinal X-X' du manche de préhension 1 de l'outil chirurgical formé, la rainure longitudinale principale 8 entraîne mécaniquement en rotation l'ailette longitudinale principale 8A, qui est de forme complémentaire, en entrant en contact avec cette dernière. La rainure longitudinale principale 8 s'étend par exemple radialement à l'axe longitudinal X-X', le long d'un plan qui est à la fois orthogonal au plan de joint Pⱼ et à la fois comprend l'axe longitudinal X-X'.

De manière préférentielle, l'orifice de réception 4 présente également deux rainures longitudinales annexes 9, 10 disposées symétriquement par rapport à un plan formé par la rainure longitudinale principale 8, les rainures longitudinales annexes 9, 10 étant conçues pour coopérer chacune avec une ailette longitudinale annexe 9A, 10A de l'embout de travail 2 afin de solidariser en rotation ledit embout de travail 2 par rapport audit manche de préhension 1 autour de l'axe longitudinal X-X'. Le plan formé par la rainure longitudinale principale 8 incluant avantageusement l'axe longitudinal X-X' et étant avantageusement orthogonal au plan de joint Pⱼ, le ailettes annexes 9A, 10A s'étendent ainsi préférentiellement le long dudit plan de joint Pⱼ, tel qu'illustré aux figures, afin de former une orifice de réception 4 en « T ». Alternativement, les rainures longitudinales 8, 9, 10 de l'orifice de réception 4 pourront par exemple être disposées en « *Y* ». L'orifice de réception 4 pourra également présenter davantage d'ailettes longitudinales, afin de présenter une forme en croix, ou en étoile. L'utilisation de rainures principales et annexes peut également s'appliquer au cas préférentiel représenté aux figures 11 à 13, ces rainures étant alors orthogonales le long de l'axe orthogonal Z-Z' et non longitudinales le long de l'axe longitudinal X-X' (non illustré aux figures).

De même, pour la variante alternative de l'invention illustrée par exemple aux figures 11 à 13, l'orifice de réception 4 présente une section dont la forme permet de bloquer en rotation l'embout de travail 2 par rapport audit manche de préhension 1 autour de l'axe orthogonal Z-Z', tout en autorisant le coulissement dudit embout de travail 2 par rapport au manche de préhension 1 le long de l'axe orthogonal Z-Z'. L'orifice de réception 4 n'autorise sensiblement ainsi, selon cette configuration, qu'un seul degré de liberté à l'embout de travail 2 lorsque ce dernier est inséré au sein dudit orifice de réception 4.

De préférence, l'orifice de réception 4 forme une empreinte femelle à pans 25, par exemple une empreinte hexagonale, qui est conçue pour coopérer avec une forme mâle à pans 26 correspondante de l'embout de travail 2 afin de solidariser en rotation ledit embout de travail 2 par rapport audit manche de préhension 1 autour de l'axe orthogonal Z-Z' (tel qu'illustré aux figures 11 à 13), ou respectivement autour de l'axe longitudinal X-X' selon la variante envisagée (ce qui n'est pas représenté aux figures).

Quelle que soit la forme de l'orifice de réception 4, l'embout de travail 2 présente une forme adaptée correspondante, de sorte qu'il puisse être inséré dans ledit orifice de réception 4.

Ces formes préférentielles de l'orifice de réception 4 permettent avantageusement de réduire le jeu nécessaire au bon coulissement de l'embout de travail 2 au sein dudit orifice de réception 4, de sorte que l'assemblage dudit embout de travail 2 avec le manche de préhension 1 est particulièrement précis et fiable.

Pour le cas préférentiel représenté aux figures 1 à 3, l'orifice de réception 4 présentant ainsi trois rainures longitudinales 8, 9, 10 ou plus, il est dès lors possible d'y insérer un embout de travail 2 présentant un nombre inférieur d'ailettes longitudinales correspondantes. En particulier, il est possible d'envisager avantageusement l'insertion dans l'orifice de réception 4 d'un embout de travail 2 ne présentant que deux, ou une seule ailette longitudinale. A titre d'exemple, il sera possible d'insérer des embouts de travail 2 du genre de ceux représentés aux figures 9 et 10, n'incluant chacun que les deux ailettes longitudinales annexes 9A, 10A, et étant dénués d'ailette principale 8A, notamment dans le cas où de tels embouts de travail 2 sont destinés à former avec le manche de préhension un outil chirurgical qui n'est pas particulièrement destiné à subir une rotation autour de l'axe longitudinal X-X'. De tels embouts de travail formeront avantageusement respectivement le guide de mise en place de broches 2B et le guide de perçage 2C, par exemple pour la mise en place d'une agrafe. Dans ce cas préférentiel, l'embout de travail 2, et en particulier les ailettes longitudinales annexes 9A, 10A pourront être munies de moyens d'adhérence 11 conçus pour que, ledit embout de travail 2 étant introduit dans l'orifice de réception 4, les moyens d'adhérence 11 permettent de bloquer ou au moins durcir le coulissement dudit embout de travail 2 au sein dudit orifice de réception 4, de manière à solidariser le manche de préhension 1 avec ledit embout de travail 2. De tels moyens d'adhérence 11 pourront être formés par exemple par des godrons, ou des picots, disposés sur le bord ou la tranche des ailettes longitudinales annexes 9A, 10A, tel qu'illustré aux figures 9 et 10.

Quelle que soit la variante de l'invention envisagée, de préférence, l'orifice de réception 4 présente une butée de fin de course de coulissement conçue pour arrêter en translation l'embout de travail 2 le long de l'axe longitudinal X-X', ou respectivement le long de l'axe orthogonal Z-Z', lorsque ce dernier est inséré dans ledit orifice de réception 4 à une profondeur déterminée. La butée de fin de course de coulissement est par exemple formée par le contour de l'entrée 12 de l'orifice de réception 4, contre lequel des ergots 13 ou picots de l'embout de travail 2 (tel qu'illustré à la figure 8), par exemple disposés sur au moins l'une des, ou les deux, ailettes longitudinales annexes 9A, 10A, sont destinés à venir en butée. Alternativement, la butée de fin de course est formée par le fond de l'orifice de réception 4 (non illustré). Cette configuration préférentielle permet de renforcer encore la précision du montage de l'embout de travail 2 au sein de l'orifice de réception 4.

Le manche de préhension 1 de l'invention comprend un levier de blocage 14 du coulissement de l'embout de travail 2 dans l'orifice de réception 4.

Le levier de blocage 14 permet au chirurgien de bloquer ou débloquer le coulissement de l'embout de travail dans l'orifice de réception 4, par exemple à l'aide d'une simple pression sur ledit levier 14. En supprimant au moins le degré de liberté en translation le long de l'axe longitudinal X-X' de l'embout de travail 2 dans par rapport au corps principal 3 du manche de préhension 1, le levier de blocage 14 permet de solidariser ledit embout de travail 2 avec ledit manche de préhension 1, de manière à former l'outil chirurgical. Il permet également de libérer à nouveau ledit degré de liberté en translation de l'embout de travail 2 par rapport au corps principal 3 afin de libérer ledit embout de travail 2 du manche de préhension 1.

De manière avantageuse, la présence du levier de blocage 14 permet de réaliser un orifice de réception 4 très coulissant, avec un jeu minimal, de manière à rendre l'insertion de l'embout de travail 2 particulièrement aisée. L'effort longitudinal exercé sur l'embout de travail 2 est alors avantageusement repris totalement ou en partie par le levier de blocage 14, optionnellement associé à la fin de course de coulissement décrite ci-avant. Le placement et le maintien en position de l'embout de travail 2 est ainsi particulièrement précis et solide, de sorte que l'outil chirurgical formé est tout à fait fiable tout en étant pratique.

Selon l'invention, le levier de blocage 14 est monté à rotation sur le corps principal 3 autour d'un axe de rotation Y-Y' de manière à pouvoir être basculé entre :
o une orientation de blocage dans laquelle ledit levier bloque le coulissement de l'embout de travail 2 (représentée à la figure 1),
∘ une orientation de libération dans laquelle il autorise le coulissement de l'embout de travail 2 dans l'orifice de réception 4.

Le levier de blocage 14 de l'invention est conçu pour adopter un mouvement de bascule autour de l'axe de rotation Y-Y', de sorte par exemple qu'une mise en mouvement vers le bas sur l'une de ses extrémités permet de mettre en mouvement l'autre de ses extrémités vers le haut, et inversement, au cours d'une rotation autour de l'axe de rotation Y-Y'. De façon préférentielle, le levier de blocage 14 s'étend entre une extrémité de blocage 17 de l'embout de travail 2 et une extrémité de manœuvre 18 manuelle dudit levier de blocage 14. Ainsi, de préférence, une action de la part du chirurgien sur l'extrémité de manoeuvre 18 permet de faire basculer le levier de blocage 14, un tel mouvement étant transmis par le corps dudit levier de blocage 14 à l'extrémité de blocage 17. Préférentiellement, l'axe de rotation Y-Y' est ainsi situé entre l'extrémité de blocage 17 et ladite extrémité de manœuvre 18. De préférence, une pression sur l'extrémité de manœuvre 18 permet de faire basculer le levier de blocage 14 vers son orientation de libération. Alternativement bien sûr, sans sortir du cadre de l'invention, un soulèvement de l'extrémité de manœuvre 18 permet de faire basculer le levier de blocage 14 vers son orientation de libération.

De préférence, de l'extrémité de blocage 17 à l'extrémité de manœuvre 18, le levier est disposé sensiblement parallèlement à l'axe longitudinal X-X', en orientation de blocage ou en orientation de libération. L'axe de rotation Y-Y' est avantageusement sensiblement orthogonal (tel qu'illustré aux figures 11 à 13), ou sensiblement ortho-radial (tel qu'illustré aux figures 1 à 3) par rapport à l'axe longitudinal X-X'.

On entend par « *ortho-radial* » que l'axe de rotation Y-Y' est sensiblement coaxial à une tangente d'un cercle lui-même coaxial à l'axe longitudinal X-X'. Le levier de blocage 14 peut ainsi être actionné par le chirurgien lorsque ce dernier met en rotation ledit levier de blocage 14 par exemple de quelques degrés ou dizaines de degrés, autour de l'axe de rotation Y-Y'. Dans le cas préférentiel des figures 11 à 13, l'axe de rotation Y-Y' est avantageusement parallèle à l'axe orthogonal Z-Z', de sorte que les trois axes X-X', Y-Y' et Z-Z' sont appartiennent géométriquement au plan de joint Pⱼ.

Selon l'invention, l'orientation de libération du levier 14 permet de libérer le coulissement de l'embout de travail 2 notamment dans le cas où le coulissement ce dernier était à l'état bloqué par ledit levier 14. En orientation de libération, l'embout de travail 2 peut parfaitement coulisser le long de l'axe longitudinal X-X', ou respectivement le long de l'axe orthogonal Z-Z', dans l'orifice de réception 4, et en être librement extrait, ou au contraire y être librement inséré.

En orientation de blocage, une portion ou une extrémité du levier vient de préférence en contact avec l'embout de travail 2 ou une portion de ce dernier, afin de bloquer le coulissement dudit embout de travail 2 dans l'orifice de réception 4. De préférence, le blocage du coulissement de l'embout de travail 2 par le levier de blocage 14 ne peut être effectué que si l'embout de travail 2 est inséré au sein de l'orifice de réception jusqu'à être sensiblement en butée axiale contre la butée de fin de course, ou au moins jusqu'à être dans une position voisine de la position dans laquelle il est en butée de fin de course. Cette position « *en fin de course* » correspond avantageusement à la position relative de l'embout de travail 2 et du corps principal 3 lorsque l'outil chirurgical est assemblé.

La libération ou le blocage de l'embout de travail 2 est par conséquent particulièrement aisée, dans la mesure où une simple pression sur ledit, ou mise en mouvement dudit, levier de blocage 14 permet la libération et/ou le blocage dudit embout de travail 2 au sein de la poignée de préhension 1.

De façon préférentielle, tel qu'illustré aux figures, le manche de préhension 1 comprend une gorge 19 ménagée dans le corps principal 3 et s'étendant de façon longitudinale, le levier de blocage 14 étant disposé au sein de ladite gorge 19 de manière à ne pas dépasser du contour extérieur du corps principal 3 sur au moins un tiers de la longueur dudit levier de blocage 14 lorsque ce dernier est en orientation de blocage.

On entend par « *contour extérieur »* le contour virtuel du corps principal 3, comme si ce dernier étant dépourvu de toute encoche, cavité, gorge, ou sillon. De préférence, le contour extérieur du corps principal 3 présente la forme d'un ellipsoïde de révolution autour de l'axe longitudinal X-X'. La gorge 19 forme préférentiellement un sillon, par exemple radial et longitudinal par rapport à l'axe longitudinal X-X' du corps principal 3, et au sein duquel tout ou partie du levier de blocage 14 est noyé, ou intégré.

Selon cette configuration préférentielle, le levier de blocage 14 ne fait pas saillie, ou peu saillie, du corps principal 3, de sorte qu'il ne fait pas obstacle à l'ergonomie de la préhension par le chirurgien du corps principal 3. De préférence, en orientation de blocage, le levier de blocage 14 suit la courbe extérieure du contour de la gorge 19, et donc du corps principal 3, par exemple depuis l'extrémité de blocage 17 jusqu'à la moitié, deux tiers, ou trois quarts de la longueur dudit levier de blocage 14 en direction de l'extrémité de manœuvre 18. De ce fait, cette dernière fait au contraire saillie du corps principal 3 de manière à pouvoir être accessible pour être manœuvrée. Le manche de préhension 1 comprend avantageusement une concavité de manoeuvre 20 ménagée dans le corps principal 3, au sein de laquelle l'extrémité de manœuvre 18 du levier de blocage 14 fait saillie. Ainsi, le levier de blocage 14 fait saillie de la concavité de manoeuvre 20, de préférence au voisinage de son extrémité de manœuvre 18, afin de pouvoir être manœuvré facilement. De préférence, le levier de blocage 14 fait suffisamment peu saillie de la concavité de manœuvre 20 pour ne pas dépasser le contour extérieur du corps principal 3. Une telle conception rend le manche de préhension 1 parfaitement confortable et ergonomique, tout en permettant une manœuvre facilitée du levier de blocage 14.

Le manche de préhension 1 de l'invention comprend également un pivot élastique 15, 16 par l'intermédiaire duquel le levier de blocage 14 est monté à rotation sur le corps principal 3, le pivot élastique 15, 16 étant conçu pour ramener de lui-même le levier de blocage 14 en orientation de blocage lorsque ce dernier est en orientation de libération. Le pivot élastique 15, 16 cumule les fonctions de :
- liaison de pivotement du levier de blocage 14 par rapport au corps principal 3 autour de l'axe de rotation Y-Y',
- moyen de rappel automatique du levier de blocage 14 vers son orientation de blocage.

De cette façon, lorsque l'embout de travail 2 est dans la position adéquate au sein de l'orifice de réception 4, et est en particulier au voisinage de la position de fin de course, le levier de blocage 14 s'oriente de lui-même en position de blocage afin de bloquer le coulissement de l'embout de travail 2. La solidarisation de l'embout de travail 2 avec le manche de préhension 1 est ainsi tout à fait aisée.

De préférence, le levier de blocage 14 revenant de lui-même en orientation de blocage, il est en orientation de blocage en l'absence d'action ou de manœuvre du chirurgien sur ledit levier de blocage 14. De préférence, l'embout de travail 2 et le levier de blocage 14 sont conformés pour que l'insertion de l'embout de travail 2 dans l'orifice de réception 4 entraîne une modification de l'orientation du levier de blocage 14 vers sa position de libération, jusqu'à ce que l'embout de travail 2 soit inséré suffisamment profondément dans l'orifice de réception 4, jusqu'à une position dite « *de fin de course* » dans laquelle levier de blocage 14 bascule de lui-même sous l'action du pivot élastique 15, 16 vers son orientation de blocage, et bloque donc automatiquement le coulissement de l'embout de travail 2 sans action ou manœuvre du chirurgien sur ledit levier de blocage 14.

De préférence, le levier de blocage 14 est pourvu d'une pente d'entraînement 23 dudit levier de blocage 14 vers son orientation de libération, ladite pente d'entraînement 23 étant à cet effet conçue pour être entraînée par une pente de soulèvement 24 réciproque de l'embout de travail 2 lors de l'insertion dudit embout de travail 2 (tel qu'illustré aux figures 5 à 7). La pente de soulèvement 24 est préférentiellement formée par un bord extrémal de l'ailette longitudinale principale 8A de l'embout de travail (tel qu'illustré par exemple à la figure 4).

Pour libérer l'embout de travail 2, le chirurgien manœuvre de préférence le levier de blocage 14 de façon à l'orienter vers son orientation de libération, ce qui libère l'embout de travail 2, et permet de le faire coulisser hors de l'orifice de réception 4.

Ainsi, le montage et le démontage de l'outil chirurgical formé par la poignée de préhension 1 de l'invention est particulièrement rapide et facile.

Selon une caractéristique importante de l'invention, le corps principal 3, le levier de blocage 14 et le pivot élastique 15, 16 viennent de matière les uns avec les autres de manière à former une pièce d'un seul tenant. En l'espèce, le manche de préhension 1 de l'invention constitue une seule pièce monobloc, réalisée avantageusement dans une unique matière travaillée pour obtenir à la fois le corps principal 3, le levier de blocage 14 et le pivot élastique 15, 16. Le manche de préhension 1 de l'invention n'est pas, en particulier, le résultat de l'assemblage de plusieurs pièces. Cette conception permet au manche de préhension 1 de l'invention d'être particulièrement facile à fabriquer, par exemple fabriqué par moulage d'une préforme du manche de préhension 1, et usinage de ladite préforme du manche de préhension 1 afin de former à la fois le corps principal 3, le pivot élastique 15, 16 et le levier de blocage 14. De préférence, le manche de préhension 1 est fabriqué à l'aide d'une unique opération de moulage, c'est-à-dire que le matériau destiné à former le manche de préhension 1 dans son ensemble est inséré dans un moule, par exemple par injection ou coulée, et que le manche de préhension est formé et fini à l'ouverture du moule. Ainsi, le manche de préhension 1 ne nécessite avantageusement pour sa fabrication aucun usinage complémentaire au moulage.

Le matériau utilisé pour une telle fabrication doit être choisi pour être à !a fois suffisamment élastique pour la formation du pivot élastique 15, 16, et à la fois suffisamment résistant pour que le corps principal 3 permette une préhension solide et sûre de l'outil chirurgical. De plus, le matériau utilisé est préférentiellement moulable ou injectable pour permettre la fabrication du manche de préhension 1 selon les modalités précédentes. Enfin, le matériau doit avantageusement pouvoir être stérilisé, dans la mesure où le manche de préhension sera utilisé à des fins chirurgicales. De préférence, par conséquent, le manche de préhension 1 est réalisé en une seule pièce d'un seul tenant en matériau polymère, par exemple du PEEK, un tel matériau remplissant avantageusement tous les critères précédents. Alternativement, un alliage métallique du genre inox ou un matériau composite pourront être utilisés.

De cette manière, les polymères étant généralement relativement peu coûteux à mettre en œuvre, il est envisageable de réaliser un manche de préhension 1 jetable et/ou recyclable, et/ou à usage unique. Le manche de préhension peut par exemple être fourni sous la forme d'un kit stérilisé, le kit comprenant également des embouts de travail 2 jetables ou non, le kit étant utilisable pour une opération chirurgicale donnée, et étant destiné à être jeté ou recyclé à la fin de ladite opération chirurgicale. De cette manière la bonne stérilisation de l'environnement chirurgicale est assurée.

De façon préférentielle, l'axe de rotation Y-Y' est situé entre l'extrémité de blocage 17 et ladite extrémité de manœuvre 18 de manière à être plus proche de l'extrémité de blocage 17 que de l'extrémité de manœuvre 18. Ainsi, à couple de rotation égal autour de l'axe de rotation Y-Y' du levier de blocage 14, il est nécessaire d'effectuer une manœuvre avec moins d'effort sur l'extrémité de manœuvre 18 que sur l'extrémité de blocage 17, pour modifier l'orientation dudit levier de blocage 14 à l'encontre de l'élasticité du pivot élastique 15, 16. La manœuvre du levier de blocage 14 est ainsi particulièrement aisée, et son caractère bloquant de l'embout de travail 2 est ainsi particulièrement fort. De préférence, l'axe de rotation Y-Y' est situé à environ un tiers de la longueur du levier de blocage 14 à partir de l'extrémité de blocage 17, tel qu'illustré aux figures.

De façon avantageuse, le pivot élastique 15, 16 relie le levier de blocage 14 à au moins une paroi de la gorge 19.

Plus précisément, tel qu'illustré aux figures, le levier de blocage 14 forme avantageusement une lame s'étendant de façon sensiblement orthogonale à i'axe de rotation Y-Y', le pivot élastique 15, 16 étant formé par deux éléments de pivot 15, 16 sensiblement hémi-cylindriques dont l'axe de hauteur est formé par l'axe de rotation Y-Y', lesdits éléments de pivot étant matérialisés de part et d'autre du levier de blocage 14 pour relier ce dernier à deux parois distinctes de la gorge 19. Ces dernières sont préférentiellement parallèles et en regard, le levier de blocage 14 étant disposé entre elles deux. Le levier de blocage 14 est ainsi positionné dans le corps principal 3 de façon symétrique, ce qui est particulièrement solide et durable. Le pivot élastique 15, 16 présente ainsi avantageusement une forme en dépouille par rapport au plan de joint Pj de façon à pouvoir être réalisé en même temps que le corps principal et que le levier de blocage 14 par moulage. L'orifice d'accueil 4 est préférentiellement quant à lui en dépouille dans une direction coaxiale à l'axe longitudinal X-X' de manière à pouvoir être modelé par un tiroir d'un moule à tiroir lors de l'opération de moulage.

De façon préférentielle, la rainure longitudinale principale 8 de l'orifice de réception 4 est alignée sensiblement dans le prolongement du levier de blocage 14, l'ailette longitudinale principale 8A étant conçue pour entrer en contact avec ledit levier de blocage 14, et en particulier avec l'extrémité de blocage 17 pour permettre le blocage dudit embout de travail 2 par ledit levier de blocage 14. La rainure longitudinale principale 8, et/ou le levier de blocage 14, et/ou les sillons 5, et/ou la gorge 19 sont ainsi préférentiellement orientés le long de plans parallèles entre eux, qui sont eux-mêmes orthogonaux au plan de joint Pⱼ, ce qui facilite la fabrication du manche de préhension 1, et notamment des étapes éventuelles de moulage.

De façon préférentielle, le levier de blocage 14 est conçu pour bloquer mécaniquement le coulissement de l'embout de travail 2 par combinaison de formes du levier de blocage 14 et de l'embout de travail 2. Une telle configuration permet ainsi un maintien en position précis de l'embout de travail 2 au sein de l'orifice de réception 4. Toutefois, il est possible d'envisager que le levier de blocage 14 bloque simplement l'embout de travail 2 par adhérence.

De manière avantageuse, le pivot élastique 15, 16 fournit un effort de rappel du levier de blocage 14 vers son orientation de blocage lorsque ledit levier de blocage 14 est écarté de son orientation de blocage, l'effort de blocage s'appliquant sur l'embout de travail 2 par l'intermédiaire du levier de blocage 14, ce dernier étant pourvu d'une surface de renvoi 27 de l'effort de rappel (tel qu'illustré à la figure 7) permettant au levier de blocage 14 de plaquer l'embout de travail 2 dans la direction de l'axe longitudinal X-X' sous l'action du pivot élastique 15, 16 contre une butée de fin de course, lorsque ledit levier de blocage 14 bloque mécaniquement le coulissement dudit embout de travail 2.

De préférence, la surface de renvoi 27 est conçue pour interagir avec une surface de réception 28 de l'embout de travail 2, lesquelles sont conçues pour être mises en contact l'une avec l'autre de manière à glisser l'une contre l'autre. La surface de renvoi 27 et la surface de réception 28 sont de préférence sensiblement parallèles entre elles lorsque l'embout de travail 2 est inséré dans l'orifice de réception 4, et sont préférentiellement inclinées par rapport à l'axe longitudinal X-X', par rapport au plan de joint Pⱼ, autour par exemple d'un axe ortho-radial à l'axe longitudinal X-X'.

L'appui glissant de la surface de renvoi 27 contre la surface de réception 28 permet de transmettre à l'embout de travail 2 une composante coaxiale à l'axe longitudinal X-X' de l'effort de rappel fourni par le pivot élastique 15, 16, de manière à pouvoir plaquer ledit embout de travail 2 contre une butée de fin de course sous l'action dudit pivot élastique 15, 16 transmise par l'intermédiaire dudit levier de blocage 14.

La butée de fin de course est avantageusement une butée axiale, permettant d'arrêter l'embout de travail 2 en translation le long de l'axe longitudinal X-X', tel que décrit ci-avant.

De préférence, tel qu'illustré aux figures, le levier de blocage 14 est pourvu d'une gâche de blocage 21 conçue pour être insérée, ou pour s'insérer d'elle-même, dans une encoche de blocage 22 de l'embout de travail 2 afin de bloquer le coulissement de ce dernier lorsque le levier de blocage 14 est en orientation de blocage. De préférence, l'encoche de blocage 22 est portée par l'ailette longitudinale principale 8A. La gâche de blocage 21 forme avantageusement également la pente d'entraînement 23 du levier de blocage 14 en rotation vers son orientation de libération. Bien entendu, la disposition inverse peut avantageusement être adoptée, dans laquelle l'embout de travail 2 comporterait une gâche de blocage et le manche de préhension comporterait une encoche de blocage correspondante.

De préférence, tel qu'illustré à la figure 7, une portion du contour extérieur de la gâche de blocage 21, en particulier l'extrémité de blocage 17 du levier de blocage 14, forme la surface de renvoi 27. De même, une portion du contour extérieur de l'encoche de blocage 22 forme la surface de réception 27. De plus, la gâche de blocage 21 comprend avantageusement une surface de retenue 29, destinée à entrer en contact avec une surface réciproque 30 de l'embout de travail 2, de manière à bloquer mécaniquement le coulissement dudit embout de travail 2 hors de l'orifice de réception 4.

De préférence, la surface de renvoi 27 est également la pente d'entraînement 23.

Ainsi, la gâche de blocage 21 et l'encoche de blocage 22 ont avantageusement une forme réciproque et complémentaire.

L'invention concerne également en tant que tel un kit d'outil chirurgical comprenant un manche de préhension 1 tel que décrit ci-avant, ainsi qu'au moins un embout de travail 2 amovible, de préférence deux embouts de travail 2 amovibles de fonction différente.

On entend par « *fonction* différente » que les deux embouts de travail 2 permettent de former avec le manche de préhension 1 respectivement un premier outil chirurgical et un deuxième outil chirurgical dont la fonction sera différente du premier outil chirurgical, c'est-à-dire que lesdits outils chirurgicaux sont destinés à effectuer des actions distinctes et non semblables, ou encore d'interagir différemment avec le milieu opératoire et avec le corps du patient. Par exemple, des embouts de travail 2 de fonction différente permettent de visser ou dévisser respectivement une première vis avec une première empreinte de tête, et une deuxième vis avec une deuxième empreinte de tête différente de la première empreinte de tête.

De préférence, les embouts de travail 2 correspondent à ceux décrits ci-avant. De manière avantageuse, conformément à ce qui précède, l'un des embouts de travail 2 est formé par un embout de tournevis 2A, un autre des embouts de travail 2 étant formé par un guide de perçage 2C, par exemple pour la mise en place d'agrafes d'ostéosynthèse et/ou de broches, ou un guide de mise en place de broches 2B.

Le kit d'outil chirurgical comprend avantageusement les broches, les vis, les agrafes, et des embouts de travail 2 supplémentaires, par exemple :
- au moins un deuxième embout de travail 2 de tournevis, lequel permet de visser une vis dont l'empreinte ou le diamètre nominal diffère de la vis qui peut être vissé par l'embout de tournevis 2A, et/ou
- un embout de travail 2 de préhension des agrafes, et/ou
- un embout de travail 2 d'impaction des agrafes dans un os du patient, et/ou
- un embout de travail 2 d'impaction des broches dans un os du patient, et/ou
- un embout de travail 2 de perçage de trous dans un os du patient.

Le kit d'outil chirurgical est de préférence jetable et/ou à usage unique, et est préférentiellement contenu dans un emballage sous atmosphère stérilisée.

L'invention concerne en outre en tant que tel un procédé de fabrication d'un manche de préhension 1 tel que décrit ci-avant. Selon l'invention, le procédé de fabrication comprend une unique étape de moulage au cours de laquelle on réalise le manche de préhension 1 d'un seul tenant dans son intégralité. En d'autres termes, le procédé de fabrication de l'invention permet de réaliser le manche de préhension 1 tel que décrit ci-avant, en une seule opération, sans autre opération de finition du genre usinage ou autre. La matière destinée à être moulée au cours du procédé de fabrication est avantageusement un polymère.

De préférence, la fabrication du manche de préhension se déroule de la manière suivante :
- on verse ou on coule ou on injecte le matériau destiné à former le manche de préhension 1 dans une machine de fabrication formée par un moule, par exemple un moule à tiroirs, de manière à ce que le matériau soit modelé par la machine de fabrication, en l'espèce par le moule,
- on ouvre le moule une fois le matériau modelé, ce dernier formant le manche de préhension 1 dans sa forme finale.

L'invention concerne enfin en tant que telle une machine de fabrication (non illustrée aux figures) d'un manche de préhension 1 tel que décrit ci-avant, et permettant de mettre en œuvre le procédé de fabrication décrit ci-avant.

De préférence, la machine de fabrication de l'invention comprend un moule comprenant une première empreinte de moule, une deuxième empreinte de moule et au moins un premier tiroir, lesquels sont destinés à former un espace clos dans lequel un matériau prévu pour former le manche de préhension 1 est destiné à être coulé. De préférence, lorsque le matériau est coulé dans le moule :
- la première empreinte de moule et la deuxième empreinte de moule sont conçues pour modeler conjointement le matériau afin de former, au moins en partie, le corps principal 3, le levier de blocage 14 et le pivot élastique 15, 16,
- le premier tiroir étant conçu pour ménager l'orifice de réception 4 au sein du corps principal 3.

La machine de fabrication de l'invention forme ainsi avantageusement un moule à tiroirs.

Au sens de l'invention, « *modeler* » signifie donner une forme par moulage à de la matière.

La première empreinte est avantageusement conçue pour modeler la partie du manche de préhension 1 s'élevant à partir du plan de joint Pⱼ dans une première direction de l'espace, la deuxième empreinte étant conçue pour modeler la partie du manche de préhension 1 s'élevant à partir du plan de joint Pⱼ dans une deuxième direction de l'espace opposée à ladite première direction de l'espace.

De préférence, la machine de fabrication comprend également un deuxième tiroir conçu pour, avec le premier tiroir, ménager l'orifice de réception 4 au sein du corps principal 3, le premier tiroir servant à ménager une première portion de l'orifice de réception 4 s'étendant à partir de l'extrémité distale 6, le deuxième tiroir servant à ménager une deuxième portion de l'orifice de réception 4 s'étendant à partir de l'extrémité proximale 7.

Lorsque le moule est fermé, c'est-à-dire lorsque la première empreinte est rapportée contre la deuxième empreinte, les tiroirs sont préférentiellement conçus pour s'étendre le long du plan de joint Pⱼ de manière à ménager l'orifice de réception 4.

La machine de fabrication de l'invention permet ainsi de réaliser le manche de préhension 1 de l'invention en une seule opération de moulage, de préférence sans usinage complémentaire, de sorte que le manche de préhension 1 de l'invention est particulièrement facile, rapide, et peu coûteux à fabriquer.

### POSSIBILITE D'APPLICATION INDUSTRIELLE

L'invention trouve son application industrielle dans la conception, la fabrication et l'utilisation de manches de préhension conçus pour recevoir un embout de travail amovible afin de former un outil chirurgical avec ce dernier, de kits d'outils chirurgicaux comprenant un tel manche de préhension, et de machines de fabrication destinées à la mise en oeuvre d'un procédé de fabrication de tels de manches de préhension.

## Revendications

1. Manche de préhension (1) conçu pour recevoir un embout de travail (2) amovible afin de former un outil chirurgical avec ce dernier, ledit manche de préhension (1) comprenant :
- un corps principal (3) s'étendant le long d'un axe longitudinal (X-X') entre une extrémité proximale (7) et une extrémité distale (6),
- un orifice de réception (4) ménagé au sein du corps principal (3) et étant conçu pour recevoir l'embout de travail (2) à coulissement,
ledit manche de préhension (1) étant **caractérisé en ce qu'**il comprend
- un levier de blocage (14) du coulissement de l'embout de travail (2) dans l'orifice de réception (4), le levier de blocage (14) étant monté à rotation sur le corps principal (3) autour d'un axe de rotation (Y-Y') de manière à pouvoir être basculé entre :
∘ une orientation de blocage dans laquelle ledit levier bloque le coulissement de l'embout de travail (2),
∘ une orientation de libération dans laquelle il autorise le coulissement de l'embout de travail (2) dans l'orifice de réception (4),
- un pivot élastique (15, 16) par l'intermédiaire duquel le levier de blocage (14) est monté à rotation sur le corps principal (3), le pivot élastique (15, 16) étant conçu pour ramener de lui-même le levier de blocage (14) en orientation de blocage lorsque ce dernier est en orientation de libération,
et **en ce que** le corps principal (3), le levier de blocage (14) et le pivot élastique (15, 16) viennent de matière les uns avec les autres de manière à former une pièce d'un seul tenant.

2. Manche de préhension (1) selon la revendication précédente, **caractérisé en ce que** le levier de blocage (14) s'étend entre une extrémité de blocage (17) de l'embout de travail (2) et une extrémité de manœuvre (18) manuelle dudit levier de blocage (14), l'axe de rotation (Y-Y') étant situé entre l'extrémité de blocage (17) et ladite extrémité de manœuvre (18) de manière à être plus proche de l'extrémité de blocage (17) que de l'extrémité de manœuvre (18).

3. Manche de préhension (1) selon l'une quelconque des revendications précédentes, **caractérisé en ce qu'**il comprend une gorge (19) ménagée dans le corps principal (3) et s'étendant de façon longitudinale, le levier de blocage (14) étant disposé au sein de ladite gorge (19) de manière à ne pas dépasser du contour extérieur du corps principal (3) sur au moins un tiers de la longueur dudit levier de blocage (14) lorsque ce dernier est en orientation de blocage.

4. Manche de préhension (1) selon l'une quelconque des revendications précédentes, **caractérisé en ce que** le levier de blocage (14) est conçu pour bloquer mécaniquement le coulissement de l'embout de travail (2) par combinaison de formes du levier de blocage (14) et de l'embout de travail (2).

5. Manche de préhension (1) selon la revendication précédente, **caractérisé en ce que** le pivot élastique (15, 16) fournit un effort de rappel du levier de blocage (14) vers son orientation de blocage lorsque ledit levier de blocage (14) est écarté de son orientation de blocage, l'effort de blocage s'appliquant sur l'embout de travail (2) par l'intermédiaire du levier de blocage (14), ce dernier étant pourvu d'une surface de renvoi (27) de l'effort de rappel permettant au levier de blocage (14) de plaquer l'embout de travail (2) dans la direction de l'axe longitudinal (X-X') sous l'action du pivot élastique (15, 16) contre une butée de fin de course, lorsque ledit levier de blocage (14) bloque mécaniquement le coulissement dudit embout de travail (2).

6. Manche de préhension (1) selon l'une quelconque des revendications précédentes, **caractérisé en ce que** l'orifice de réception (4) est ménagé au sein du corps principal (3) à partir de l'extrémité distale (6).

7. Manche de préhension (1) selon la revendication précédente, **caractérisé en ce que** l'orifice de réception (4) est conçu pour recevoir l'embout de travail (2) à coulissement le long de l'axe longitudinal (X-X'), de manière à former un outil chirurgical axial.

8. Manche de préhension (1) selon l'une quelconque des revendications 1 à 5, **caractérisé en ce que** l'orifice de réception (4) est ménagé au sein du corps principal (3), de manière à déboucher à partir de la surface externe de ce dernier, entre l'extrémité distale (6) et l'extrémité proximale (7).

9. Manche de préhension (1) selon la revendication précédente, **caractérisé en ce que** l'orifice de réception (4) est conçu pour recevoir l'embout de travail (2) à coulissement le long d'un axe orthogonal (Z-Z') orthogonal à l'axe longitudinal (X-X') de manière à former un outil chirurgical en « *T* ».

10. Manche de préhension (1) selon l'une quelconque des revendications précédentes, **caractérisé en ce qu'**il forme un manche de tournevis.

11. Manche de préhension (1) selon l'une quelconque des revendications précédentes, **caractérisé en ce qu'**il est réalisé en une seule pièce d'un seul tenant en matériau polymère et **en ce qu'**il est fabriqué à l'aide d'une unique opération de moulage.

12. Manche de préhension (1) selon l'une quelconque des revendications précédentes, **caractérisé en ce que** l'axe de rotation (Y-Y') est sensiblement orthogonal, ou sensiblement ortho-radial par rapport à l'axe longitudinal (X-X').

13. Kit d'outil chirurgical comprenant un manche de préhension (1) selon l'une quelconque des revendications précédentes, ainsi qu'au moins un embout de travail (2) amovible, de préférence deux embouts de travail (2) amovibles de fonction différente.

14. Procédé de fabrication d'un manche de préhension (1) selon l'une quelconque des revendications 1 à 12, le procédé de fabrication étant **caractérisé en ce qu'**il comprend une unique étape de moulage au cours de laquelle on réalise le manche de préhension (1) d'un seul tenant dans son intégralité.

15. Machine de fabrication d'un manche de préhension (1) selon l'une quelconque des revendications 1 à 12, ladite machine de fabrication étant **caractérisée en ce qu'**elle comprend un moule comprenant une première empreinte de moule, une deuxième empreinte de moule et au moins un premier tiroir, lesquels sont destinés à former un espace clos dans lequel un matériau prévu pour former le manche de préhension (1) est destiné à être coulé, la première empreinte de moule et la deuxième empreinte de moule étant conçues pour modeler conjointement le matériau afin de former, au moins en partie, le corps principal (3), le levier de blocage (14) et le pivot élastique (15, 16), le premier tiroir étant conçu pour ménager l'orifice de réception (4) au sein du corps principal (3), ladite machine de fabrication permettant de mettre en oeuvre le procédé de fabrication selon la revendication 14.

## Patentansprüche

1. Greifgriff (1), ausgelegt zum Aufnehmen eines abnehmbaren Arbeitsaufsatzes (2), um damit ein chirurgisches Werkzeug zu bilden, wobei der Greifgriff (1) Folgendes umfasst:
- einen Hauptkörper (3), der sich entlang einer Längsachse (X-X') zwischen einem proximalen Ende (7) und einem distalen Ende (6) erstreckt,
- eine Aufnahmeöffnung (4), die innerhalb des Hauptkörpers (3) vorgesehen und zur Aufnahme des gleitenden Arbeitsaufsatzes (2) ausgelegt ist,
wobei der Greifgriff (1) **dadurch gekennzeichnet ist, dass** er Folgendes umfasst
- einen Hebel (14) zum Blockieren des Gleitens des Arbeitsaufsatzes (2) in der Aufnahmeöffnung (4), wobei der Blockierhebel (14) um eine Drehachse (Y-Y') drehbar am Hauptkörper (3) montiert ist, sodass er gekippt werden kann zwischen:
∘ einer Blockierrichtung, in der der Hebel das Gleiten des Arbeitsaufsatzes (2) blockiert,
∘ einer Freigaberichtung, in der er das Gleiten des Arbeitsaufsatzes (2) in der Aufnahmeöffnung (4) erlaubt,
- einen elastischen Stift (15, 16), durch den der Blockierhebel (14) drehbar am Hauptkörper (3) montiert ist, wobei der elastische Stift (15, 16) derart ausgelegt ist, dass er den Blockierhebel (14) von selbst in die Blockierrichtung zurückführt, wenn dieser sich in der Freigaberichtung befindet,
und dass der Hauptkörper (3), der Blockierhebel (14) und der elastische Stift (15, 16) miteinander integral aufgebaut sind, sodass sie ein Teil in einem Stück bilden.

2. Greifgriff (1) nach dem vorhergehenden Anspruch, **dadurch gekennzeichnet, dass** sich der Blockierhebel (14) zwischen einem Blockierende (17) des Arbeitsaufsatzes (2) und einem manuellen Betätigungsende (18) des Blockierhebels (14) erstreckt, wobei die Drehachse (Y-Y') zwischen dem Blockierende (17) und dem Betätigungsende (18) derart angeordnet ist, dass sie näher an dem Blockierende (17) als an dem Betätigungsende (18) liegt.

3. Greifgriff (1) nach einem der vorhergehenden Ansprüche, **dadurch gekennzeichnet, dass** er eine im Hauptkörper (3) vorgesehene und sich in Längsrichtung erstreckende Nut (19) umfasst, wobei der Blockierhebel (14) derart innerhalb der Nut (19) angeordnet ist, dass er sich über mindestens ein Drittel der Länge des Blockierhebels (14) nicht über die äußere Kontur des Hauptkörpers (3) hinaus erstreckt, wenn der Hebel in Blockierrichtung ist.

4. Greifgriff (1) nach einem der vorhergehenden Ansprüche, **dadurch gekennzeichnet, dass** der Blockierhebel (14) ausgelegt ist, um das Gleiten des Arbeitsaufsatzes (2) durch Kombinieren von Formen des Blockierhebels (14) und des Arbeitsaufsatzes (2) mechanisch zu blockieren.

5. Greifgriff (1) nach dem vorhergehenden Anspruch, **dadurch gekennzeichnet, dass** der elastische Stift (15, 16) eine Rückstellkraft des Blockierhebels (14) in seine Blockierrichtung bereitstellt, wenn der Blockierhebel (14) von seiner Blockierrichtung wegbewegt wird, wobei die Blockierkraft über den Blockierhebel (14) auf den Arbeitsaufsatz (2) aufgebracht wird, wobei der Hebel mit einer Rücklauffläche (27) für die Rückstellkraft versehen ist, die es dem Blockierhebel (14) ermöglicht, den Arbeitsaufsatz (2) unter der Wirkung des elastischen Stifts (15, 16) in Richtung der Längsachse (X-X') gegen einen Anschlag zu drücken, wenn der Blockierhebel (14) das Gleiten des Arbeitsaufsatzes (2) mechanisch blockiert.

6. Greifgriff (1) nach einem der vorhergehenden Ansprüche, **dadurch gekennzeichnet, dass** die Aufnahmeöffnung (4) innerhalb des Hauptkörpers (3) vom distalen Ende (6) aus vorgesehen ist.

7. Greifgriff (1) nach dem vorhergehenden Anspruch, **dadurch gekennzeichnet, dass** die Aufnahmeöffnung (4) ausgelegt ist, um den entlang der Längsachse (X-X') gleitenden Arbeitsaufsatz (2) aufzunehmen, um ein axiales chirurgisches Werkzeug zu bilden.

8. Greifgriff (1) nach einem der vorhergehenden Ansprüche 1 bis 5, **dadurch gekennzeichnet, dass** die Aufnahmeöffnung (4) innerhalb des Hauptkörpers (3) derart vorgesehen ist, dass sie sich von dessen Außenfläche aus zwischen dem distalen Ende (6) und dem proximalen Ende (7) öffnet.

9. Greifgriff (1) nach dem vorhergehenden Anspruch, **dadurch gekennzeichnet, dass** die Aufnahmeöffnung (4) ausgelegt ist, um den entlang einer orthogonal zur Längsachse (X-X') stehenden orthogonalen Achse (Z-Z') gleitenden Arbeitsaufsatz (2) aufzunehmen, um ein "*T*"-förmiges chirurgisches Werkzeug zu bilden.

10. Greifgriff (1) nach einem der vorhergehenden Ansprüche, **dadurch gekennzeichnet, dass** er einen Schraubendrehergriff bildet.

11. Greifgriff (1) nach einem der vorhergehenden Ansprüche, **dadurch gekennzeichnet, dass** er aus einem einzigen Teil in einem Stück aus Polymermaterial hergestellt ist und dass er mittels eines einzigen Formvorgangs hergestellt wird.

12. Greifgriff (1) nach einem der vorhergehenden Ansprüche, **dadurch gekennzeichnet, dass** die Drehachse (Y-Y') im Wesentlichen orthogonal oder im Wesentlichen orthoradial zur Längsachse (X-X') ist.

13. Chirurgischer Werkzeugsatz mit einem Greifgriff (1) nach einem der vorhergehenden Ansprüche sowie mindestens einem abnehmbaren Arbeitsaufsatz (2), vorzugsweise zwei abnehmbaren Arbeitsaufsätzen (2) mit unterschiedlichen Funktionen.

14. Verfahren zur Herstellung eines Greifgriffs (1) nach einem der Ansprüche 1 bis 12, wobei das Verfahren zur Herstellung **dadurch gekennzeichnet ist, dass** es einen einzigen Formschritt umfasst, bei dem der Greifgriff (1) in seiner Gesamtheit in einem Stück gefertigt wird.

15. Maschine zum Herstellen eines Greifgriffs (1) nach einem der Ansprüche 1 bis 12, wobei die Maschine zum Herstellen **dadurch gekennzeichnet ist, dass** sie eine Form umfasst, die einen ersten Formhohlraum, einen zweiten Formhohlraum und mindestens einen ersten Einschub umfasst, die dazu bestimmt sind, einen geschlossenen Raum zu bilden, in den ein zum Bilden des Greifgriffs (1) vorgesehenes Material gegossen werden soll, wobei der erste Formhohlraum und der zweite Formhohlraum ausgelegt sind, um das Material gemeinsam zu formen, um zumindest teilweise den Hauptkörper (3), den Blockierhebel (14) und den elastischen Stift (15, 16) zu bilden, wobei der erste Einschub ausgelegt ist, die Aufnahmeöffnung (4) innerhalb des Hauptkörpers (3) vorzusehen, wobei die Herstellungsmaschine es ermöglicht, dass das Herstellungsverfahren nach Anspruch 14 durchgeführt wird.

## Claims

1. A gripping handle (1) designed to receive a removable working bit (2) in order to form a surgical tool with the latter, said gripping handle (1) comprising:
- a main body (3) extending along a longitudinal axis (X-X') between a proximal end (7) and a distal end (6),
- a receiving orifice (4) formed within the main body (3) and being designed to slidingly receive the working bit (2),
said gripping handle (1) being **characterized in that** it comprises
- a lever (14) for blocking the sliding of the working bit (2) in the receiving orifice (4), the blocking lever (14) being rotatably mounted on the main body (3) about an axis of rotation (Y-Y') so as to be able to tilt between:
∘ a blocking orientation in which said lever blocks the sliding of the working bit (2),
∘ a release orientation in which it enables the sliding of the working bit (2) in the receiving orifice (4),
- an elastic pivot (15, 16) by which the blocking lever (14) is rotatably mounted on the main body (3), the elastic pivot (15, 16) being designed to bring, by itself, the blocking lever (4) back in the blocking orientation when the latter is in the release orientation,
and **in that** the main body (3), the blocking lever (14) and the elastic pivot (15, 16) are integral with each other so as to form a one single-piece part.

2. The gripping handle (1) according to the preceding claim, **characterized in that** the blocking lever (14) extends between an end (17) for blocking the working bit (2) and an end (18) for manually maneuvering said blocking lever (14), the axis of rotation (Y-Y') being located between the blocking end (17) and said maneuvering end (18) so as to be closer to the blocking end (17) than the maneuvering end (18).

3. The gripping handle (1) according to any one of the preceding claims, **characterized in that** it comprises a groove (19) formed in the main body (3) and extending longitudinally, the blocking lever (14) being disposed within said groove (19) so as not to surpass the external contour of the main body (3) over at least one-third the length of said blocking lever (14) when the latter is in the blocking orientation.

4. The gripping handle (1) according to any one of the preceding claims, **characterized in that** the blocking lever (14) is designed to mechanically block the sliding of the working bit (2) by form-fitting between the blocking lever (14) and the working bit (2).

5. The gripping handle (1) according to the preceding claim, **characterized in that** the elastic pivot (15, 16) provides a force for returning the blocking lever (14) toward its blocking orientation when said blocking lever (14) is brought away from its blocking orientation, the blocking force being applied on the working bit (2) via the blocking lever (14), the latter being provided with a surface (27) for bearing the return force allowing the blocking lever (14) to press the working bit (2) in the direction of the longitudinal axis (X-X') under the action of the elastic pivot (15, 16) against an end-of-travel stop, when said blocking lever (14) mechanically blocks the sliding of said working bit (2).

6. The gripping handle (1) according to any one of the preceding claims, **characterized in that** the receiving orifice (4) is formed within the main body (3) from the distal end (6).

7. The gripping handle (1) according to the preceding claim, **characterized in that** the receiving orifice (4) is designed to receive the working bit (2) sliding along the longitudinal axis (X-X'), so as to form an axial surgical tool.

8. The gripping handle (1) according to any one of claims 1 to 5, **characterized in that** the receiving orifice (4) is formed within the main body (3), so as to open from the outer surface of the latter, between the distal end (6) and the proximal end (7).

9. The gripping handle (1) according to the preceding claim, **characterized in that** the receiving orifice (4) is designed to receive the working bit (2) sliding along an orthogonal axis (Z-Z') orthogonal to the longitudinal axis (X-X') so as to form a « T »-shaped surgical tool.

10. The gripping handle (1) according to any one of the preceding claims, **characterized in that** it forms a screwdriver handle.

11. The gripping handle (1) according to any one of the preceding claims, **characterized in that** it is realized in a one single-piece part from a polymer material, and **in that** it is manufactured through one single molding operation.

12. The gripping handle (1) according to any one of the preceding claims, **characterized in that** the axis of rotation (Y-Y') is substantially orthogonal, or substantially orthoradial with respect to the longitudinal axis (X-X').

13. A surgical tool kit comprising a gripping handle (1) according to any one of the preceding claims, as well as at least one removable working bit (2), preferably two removable working bits (2) having different functions.

14. A method for manufacturing a gripping handle (1) according to any one of claims 1 to 12, the manufacturing method being **characterized in that** it comprises one single molding step during which the gripping handle (1) is made in one piece in its entirety.

15. A machine for manufacturing a gripping handle (1) according to any one of claims 1 to 12, said manufacturing machine being **characterized in that** it comprises a mold comprising a first molding cavity, a second molding cavity and at least one first drawer, which are intended to form a closed space in which a material intended to form the gripping handle 1 is intended to be cast, the first mold cavity together with the second mold cavity being designed to model the material in order to form, at least partially, the main body (3), the blocking lever (14) and the elastic pivot (15, 16), the first drawer being designed to form the receiving orifice (4) within the main body (3), said manufacturing machine allowing implementing the manufacturing method according to claim 14.
